# EUROPÄISCHE PATENTSCHRIFT

(11) **EP 1 148 868 B1**
(45) Veröffentlichungstag und Bekanntmachung des Hinweises auf die Patenterteilung: **12.04.2006**
(21) Anmeldenummer: 00910607.1
(22) Anmeldetag: 25.01.2000
(51) Int. Cl.: C11D 3/382, C11D 1/62, A61K 8/41, A61K 8/97

(54) **DETERGENSGEMISCHE**
DETERGENT MIXTURES
MELANGES DETERGENTS

(30) Priorität: 04.02.1999 DE 19904513
(43) Veröffentlichungstag der Anmeldung: 31.10.2001
(73) Patentinhaber: Cognis IP Management GmbH, 40589 Düsseldorf (DE)
(72) Erfinder: WEUTHEN, Manfred, D-40764 Langenfeld (DE); FABRY, Bernd, D-41352 Korschenbroich (DE); BLASQUEZ, José, Fernandez, E-08228 Terrassa (ES); PI SUBIRANA, Rafael, E-08400 Granollers (ES)
(86) Internationale Anmeldenummer: PCT/EP2000/000531
(87) Internationale Veröffentlichungsnummer: WO 2000/045788

(56) Entgegenhaltungen:
- DE-A- 19 738 303
- DE-C- 19 730 649
- US-A- 4 919 846
- PATENT ABSTRACTS OF JAPAN vol. 008, no. 102 (C-222), 12. Mai 1984 (1984-05-12) & JP 59 016813 A (RAION KK), 28. Januar 1984 (1984-01-28)

## Beschreibung

### Gebiet der Erfindung

Die Erfindung betrifft Detergensgemische mit einem Gehalt an Esterquats und Aloe sowie die Verwendung der Gemische zur Herstellung von oberflächenaktiven Mitteln.

### Stand der Technik

Aus dem Stand der Technik sind eine Vielzahl von Tensidmischungen bekannt, die Einsatz in den unterschiedlichsten Gebieten finden. So sind aus der DE 19730649 C1 Detergensgemische bekannt die neben Esterquats Chitosane und Proteinhydrolysate enthalten. Das Patent Abstracts of Japan vol. 008, no. 102 (C-222), 12. Mai 1984 & JP 59016813 A beschreibt kosmetische Zubereitungen, die kationische Tenside und eine Aloe-Zubereitung enthalten. Die DE 197 38 303 A1 beschreibt die Verwendung von Esterquats und Aloe Vera zur Behandlung von Haaren. Im Bereich der Waschmittelrohstoffe gibt es jedoch ein gleichartiges Bedürfnis nach möglichst konzentrierten Tensidvorgemischen, die sich durch gute Avivageeigenschaften auszeichnen. Eine weitere Forderung besteht darin, daß die Produkte über eine optimale Hautverträglichkeit verfügen, so daß die Gefahr, daß selbst besonders sensibilisierte Verbraucher entweder im direkten Umgang oder indirekt über den Kontakt mit der behandelten Faser Hautirritationen erleiden, praktisch ausgeschlossen ist.

Die komplexe Aufgabe der Erfindung hat demnach darin bestanden, neue Detergensgemische für die Waschmittel industrie zur Verfügung zu stellen, die sich gleichzeitig durch eine besonders hohe Hautverträglichkeit, ein gutes Haut- und Textilreinigungs- und Wiederbenetzungsvermögen sowie ausgezeichnete Avivageeigenschaften für synthetische und natürliche Fasern auszeichnen sollten.

### Beschreibung der Erfindung

Gegenstand der Erfindung ist die Verwendung von Detergensgemische, enthaltend
(a) Esterquats und
(b) Aloe.
zur Herstellung von Avivagemitteln.

Überraschenderweise wurde gefunden, daß die erfindungsgemäßen Detergensgemische nicht nur besonders gut hautverträglich sind, sondem zudem über ein besonders hohes Reinigungsvermögen für Textilien verfügen. Textilien verleihen sie ferner nicht nur einen angenehmen Weichgriff, sie erniedrigen auch die statische Aufladung zwischen den Fasern. Die Mittel eignen sich insbesondere zur Herstellung von Avivagemitteln.

### Esterquats

Unter der Bezeichnung "Esterquats" (Komponente a) werden im allgemeinen quatemierte Fettsäuretri-ethanolaminestersalze verstanden. Es handelt sich dabei um bekannte Stoffe, die man nach den einschlägigen Methoden der präparativen organischen Chemie erhalten kann. In diesem Zusammenhang sei auf die Intemationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Übersichten zu diesem Thema sind beispielsweise von R.Puchta et al. in **Tens. Surf. Det., 30, 186 (1993),** M.Brock in **Tens. Surf. Det. 30, 394 (1993),** R.Lagerman et al. in **J. Am. Oil. Chem. Soc., 71, 97 (1994)** sowie I.Shapiro in **Cosm.Toil.109, 77 (1994)** erschienen.

Die quatemierten Fettsäuretriethanolaminestersalze folgen der Formel **(I)**, 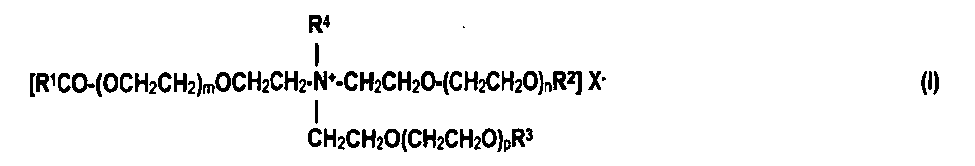 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprin-säure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäure-reiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestem mit einem durchschnittlichen Veresterungsgrad von 1,5 bis 1,9 dar und leiten sich von technischer C_{16/18}- Talg- bzw. Palmfettsäure (lodzahl 0 bis 40) ab. Aus anwendungstechnischer Sicht haben sich quaternierte Fettsäuretriethanolaminestersalze der Formel (I) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quatemierten Fettsäuretriethanolaminestersalzen kommen als Esterquats ferner auch qua-temierte Estersalze von Fettsäuren mit Diethanolalkylaminen der Formel **(II)** in Betracht, 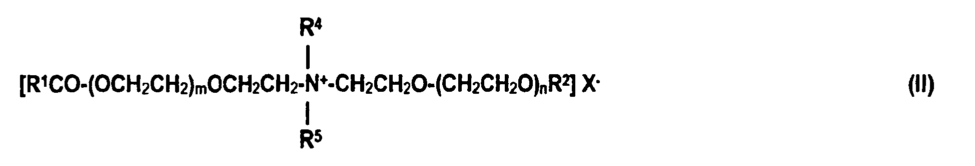 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen, 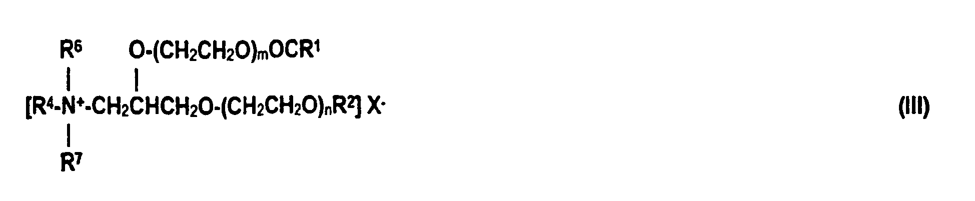 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.-%iger alkoholischer Lösungen in den Handel, die bei Bedarf problemlos mit Wasser verdünnt werden können. Es ist ferner ebenfalls möglich, die Esterquats zusammen mit Fettalkoholen in Form von Schuppen einzusetzen, wie dies beispielsweise in der Deutschen Patentschrift **DE-C1 4308794** (Henkel) beschrieben wird.

### Aloe

Unter Aloe oder dem synonymen Begriff Atoin versteht man den eingedickten Saft der Blätter von Aloe-Arten (Liliaceae), hauptsächlich von Aloe vera (Curacao-Aloe) und Aloe ferox bzw. Aloe africans (Kap-Aloe). Der Saft enthält neben Harzen, Emodin, etherischen Ölen vor allem etwa 5 bis 25 Gew.-% des Anthonderivats Aloin. Weitere Bestandteile sind das Pyronderivat Aloenin, verschiedene sich vom Anthracenon ableitende Aloesaponole sowie das Chromanonderivat Aloesin. Eine Übersicht zu diesem Thema ist in **PhiuZ, 13, 172** (1984) erschienen.

### Weitere Tenside

Die erfindungsgemäßen Detergensgemische können weitere anionische, nichtionische kationische und/oder amphotere bzw. amphotere Tenside enthalten, deren Anteil an den Mitteln üblicherweise bei etwa 1 bis 25 und vorzugsweise 5 bis 15 Gew.-% liegt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinether-sulfate, Fettsäureethersulfate, Hydroxymischethersulfate, Monoglycerid-(ether)sulfate, Fettsäureamid-(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)-yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, beispielsweise vom Typ des Dimethyldistearylammoniumchlorids. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.),** "Katalysatoren, **Tenside und Mineralöladditive", Thieme Verlag, Stuttgart, 1978, S. 123-217** verwiesen.

### Detergensgemische

In einer bevorzugten Ausführungsform der Erfindung werden die Detergensgemische in Form von wäßrigen Zubereitungen mit einem Feststoffgehalt im Bereich von 15 bis 70, vorzugsweise 25 bis 50 und insbesondere 35 bis 45 Gew.-% eingesetzt. Vorzugsweise weisen die Mittel die folgende Zusammensetzung auf:
(a) 1 bis 50, vorzugsweise 5 bis 40 Gew.-% Esterquats,
(b) 1 bis 10, vorzugsweise 2 bis 5 Gew.-% Aloe und
(c) 0 bis 25, vorzugsweise 1 bis 15 Gew.-% weitere Tenside,
mit der Maßgabe, daß sich die Gewichtsangaben mit Wasser sowie gegebenenfalls weiteren Hilfs- und Zusatzstoffen zu 100 Gew.-% ergänzen.

### Gewerbliche Anwendbarkeit

Die erfindungsgemäßen Detergensgemische verfügen über ein ausgezeichnetes Reinigungsvermögen und verleihen synthetischen wie natürlichen Fasern einen angenehmen Weichgriff; zudem emiedrigen sie die elektrostatische Aufladung zwischen den Fasern und verbessern deren Wieder-benetzbarkeit. Ein Gegenstand der Erfindung betrifft daher die Verwendung der Gemische zur Herstellung von Avivagemitteln.

### Wasch-, Spül-, Reinigunas- und Avivagemittel

Sofern die erfindungsgemäßen Detergensgemische als Wasch-, Spül-, Reinigungs- oder Avivagemittel ("Softener") dienen, liegen sie üblicherweise in flüssiger Form vor; zur Herstellung von Pulverwaschmitteln können die wäßrigen Mischungen in der Folge getrocknet werden. Flüssige Zubereitungen können einen nicht wäßrigen Anteil im Bereich von 5 bis 50 und vorzugsweise 15 bis 35 Gew.-% aufweisen. Im einfachsten Fall handelt es sich um wäßrige Lösungen der genannten Mischungen. Bei den Flüssigwaschmitteln kann es sich aber auch um im wesentlichen wasserfreie Mittel handeln. Dabei bedeutet "im wesentlichen wasserfrei" im Rahmen dieser Erfindung, daß das Mittel vorzugsweise kein freies, nicht als Kristallwasser oder in vergleichbarer Form gebundenes Wasser enthält. In einigen Fällen sind geringe Menge an freiem Wasser tolerierbar, insbesondere in Mengen bis zu 5 Gew.-%. Die im Detergensbereich eingesetzten Mittel können weitere typische Inhaltsstoffe, wie beispielsweise Builder, Bleichmittel, Bleichaktivatoren, Lösungsmittel, Waschkraftverstärker, Enzyme, Enzymstabilisatoren, Viskositätsregulatoren, Vergrauungsinhibitoren, optische Aufheller, Soil repellants, Schauminhibitoren, anorganische Salze sowie Duft- und Farbstoffe enthalten.

Geeignete flüssige Builder sind Ethylendiamintetraessigsäure, Nitrilotriessigsäure, Citronensäure sowie anorganische Phosphonsäuren, wie z.B. die neutral reagierenden Natriumsalze von 1-Hydroxyethan-1,1,-diphosphonat, die in Mengen von 0,5 bis 5, vorzugsweise 1 bis 2 Gew.-% zugegen sein können. Als **feste Builder** wird insbesondere feinkristalliner, synthetisches und gebundenes Wasser enthaltender Zeolith wie Zeolith NaA in Waschmittelqualität eingesetzt. Geeignet sind jedoch auch Zeolith NaX sowie Mischungen aus NaA und NaX. Der Zeolith kann als sprühgetrocknetes Pulver oder auch als ungetrocknete, von ihrer Herstellung noch feuchte, stabilisierte Suspension zum Einsatz kommen. Für den Fall, daß der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen oder ethoxylierte Isotridecanole. Geeignete Zeolithe weisen eine mittlere Teilchengröße von weniger als 10 µm (Volumenverteilung; Meßmethode: Coulter Counter) auf und enthalten vorzugsweise 18 bis 22, insbesondere 20 bis 22 Gew.-% an gebundenem Wasser. Geeignete Substitute bzw. Teilsubstitute für Zeolithe sind kristalline, schichtförmige Natriumsilicate der allgemeinen Formel NaMSiₓO₂ₓ₊₁·yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilicate werden beispielsweise in der europäischen Patentanmeldung **EP 0164514 A** beschrieben. Bevorzugte kristalline Schichtsilicate sind solche, in denen M in der allgemeinen Formel für Natrium steht und x die Werte 2 oder 3 annimmt. Insbesondere sind sowohl β- als auch γ-Natriumdisilicate Na₂Si₂O₅·yH₂O bevorzugt, wobei β-Natriumdisilicat beispielsweise nach dem Verfahren erhalten werden kann, das in der intemationalen Patentanmeldung **WO 91/08171** beschrieben ist. Die erfindungsgemäßen Pulverwaschmittel enthalten als feste Builder vorzugsweise 10 bis 60 Gew.-% Zeolith und/oder kristalline Schichtsilicate, wobei Mischungen von Zeolith und kristallinen Schichtsilicaten in einem beliebigen Verhältnis besonders vorteilhaft sein können. Insbesondere ist es bevorzugt, daß die Mittel 20 bis 50 Gew.-% Zeolith und/oder kristalline Schichtsilicate enthalten. Besonders bevorzugte Mittel enthalten bis 40 Gew.-% Zeolith und insbesondere bis 35 Gew.-% Zeolith, jeweils bezogen auf wasserfreie Aktivsubstanz. Weitere geeignete Inhaltsstoffe der Mittel sind wasserlösliche amorphe Silicate; vorzugsweise werden sie in Kombination mit Zeolith und/oder kristallinen Schichtsilicaten eingesetzt. Insbesondere bevorzugt sind dabei Mittel, welche vor allem Natriumsilicat mit einem molaren Verhältnis (Modul) Na₂O : SiO₂ von 1:1 bis 1:4,5, vorzugsweise von 1:2 bis 1:3,5, enthalten. Der Gehalt der Mittel an amorphen Natriumsilicaten beträgt dabei vorzugsweise bis 15 Gew.-% und vorzugsweise zwischen 2 und 8 Gew.-%. Auch Phosphate wie Tripolyphosphate, Pyrophosphate und Orthophosphate können in geringen Mengen in den Mitteln enthalten sein. Vorzugsweise beträgt der Gehalt der Phosphate in den Mitteln bis 15 Gew.-%, jedoch insbesondere 0 bis 10 Gew.-%. Außerdem können die Mittel auch zusätzlich Schichtsilicate natürlichen und synthetischen Ursprungs enthalten. Derartige Schichtsilicate sind beispielsweise aus den Patentanmeldungen **DE 2334899 B, EP 0026529 A** und **DE 3526405 A** bekannt. Ihre Verwendbarkeit ist nicht auf eine spezielle Zusammensetzung bzw. Strukturformel beschränkt. Bevorzugt sind hier jedoch Smectite, insbesondere Bentonite. Geeignete Schichtsilicate, die zur Gruppe der mit Wasser quellfähigen Smectite zählen, sind z.B. solche der allgemeinen Formeln

(OH)₄Si_{8-y}Al_{y}(MgₓAl₄₋ₓ)O₂₀ Montmorrilonit

(OH)₄Si_{8-y}Al_{y}(Mg_{6-z}Li_{z})O₂₀ Hectorit

(OH)₄Si_{8-y}Al_{y}(Mg_{6-z} Al_{z})O₂₀ Saponit

mit x = 0 bis 4, y = 0 bis 2, z = 0 bis 6. Zusätzlich kann in das Kristallgitter der Schichtsilicate gemäß den vorstehenden Formeln geringe Mengen an Eisen eingebaut sein. Ferner können die Schichtsilicate aufgrund ihrer ionenaustauschenden Eigenschaften Wasserstoff-, Alkali-, Erdalkaliionen, insbesondere Na⁺ und Ca²⁺ enthalten. Die Hydratwassermenge liegt meist im Bereich von 8 bis 20 Gew.-% und ist vom Quellzustand bzw. von der Art der Bearbeitung abhängig. Brauchbare Schichtsilicate sind beispielsweise aus **US 3,966,629, US 4,062,647, EP 0026529 A** und **EP 0028432 A** bekannt. Vorzugsweise werden Schichtsilicate verwendet, die aufgrund einer Alkalibehandlung weitgehend frei von Calciumionen und stark färbenden Eisenionen sind. Brauchbare organische Gerüstsubstanzen sind beispielsweise die bevorzugt in Form ihrer Natriumsalze eingesetzten Polycarbonsäuren, wie Citronensäure, Adipinsäure, Bemsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Geeignete polymere Polycarboxylate sind beispielsweise die Natriumsalze der Polyacrylsäure oder der Polymethacrylsäure, beispielsweise solche mit einer relativen Molekülmasse von 800 bis 150000 (auf Säure bezogen). Geeignete copolymere Polycarboxylate sind insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 5000 bis 200000, vorzugsweise 10000 bis 120000 und insbesondere 50000 bis 100000. Der Einsatz polymerer Polycarboxylate ist nicht zwingend erforderlich. Falls jedoch polymere Polycarboxylate eingesetzt werden, so sind Mittel bevorzugt, welche biologisch abbaubare Polymere, beispielsweise Terpolymere, die als Monomere Acrylsäure und Maleinsäure bzw. deren Salze sowie Vinylalkohol bzw. Vinylalkohol-Derivate oder die als Monomere Acrylsäure und 2-Alkylallylsulfonsäure bzw. deren Salze sowie Zuckerderivate enthalten. Insbesondere sind Terpolymere bevorzugt, die nach der Lehre der deutschen Patentanmeldungen **DE 4221381 A** und **DE 4300772 A** erhalten werden. Weitere geeignete Buildersubstanzen sind Polyacetale, welche durch Umsetzung von Dialdehyden mit Polyolcarbonsäuren, welche 5 bis 7 Kohlenstoffatome und mindestens 3 Hydroxylgruppen aufweisen, beispielsweise wie in der europäischen Patentanmeldung **EP 0280223 A** beschrieben erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Unter den als **Bleichmittel** dienenden, in Wasser Wasserstoffperoxid liefemden Verbindungen haben das Natriumperborat-Tetrahydrat und das Natriumperborat-Monohydrat eine besondere Bedeutung. Weitere Bleichmittel sind beispielsweise Peroxycarbonat, Citratperhydrate sowie Salze der Persäuren, wie Perbenzoate, Peroxyphthalate oder Diperoxydodecandisäure. Sie werden üblicherweise in Mengen von 8 bis 25 Gew.-% eingesetzt. Bevorzugt ist der Einsatz von Natriumperborat-Monohydrat in Mengen von 10 bis 20 Gew.-% und insbesondere von 10 bis 15 Gew.-%. Durch seine Fähigkeit, unter Ausbildung des Tetrahydrats freies Wasser binden zu können, trägt es zur Erhöhung der Stabilität des Mittels bei.

Um beim Waschen bei Temperaturen von 60°C und darunter eine verbesserte Bleichwirkung zu erreichen, können **Bleichaktivatoren** in die Präparate eingearbeitet werden. Beispiele hierfür sind mit Wasserstoffperoxid organische Persäuren bildende N-Acyl- bzw. O-Acyl-Verbindungen, vorzugsweise N,N'-tetraacylierte Diamine, ferner Carbonsäureanhydride und Ester von Polyolen wie Glucosepentaacetat. Der Gehalt der bleichmittelhaltigen Mittel an Bleichaktivatoren liegt in dem üblichen Bereich, vorzugsweise zwischen 1 und 10 Gew.-% und insbesondere zwischen 3 und 8 Gew.-%. Besonders bevorzugte Bleichaktivatoren sind N,N,N',N'-Tetraacetylethylendiamin und 1,5-Diacetyl-2,4-dioxo-hexahydro-1,3,5-triazin.

Als organische **Lösungsmittel** kommen beispielsweise mono- und/oder polyfunktionelle Alkohole mit 1 bis 6 Kohlenstoffatomen, vorzugsweise mit 1 bis 4 Kohlenstoffatomen in Frage. Bevorzugte Alkohole sind Ethanol, 1,2-Propandiol, Glycerin sowie deren Gemische. Die Mittel enthalten vorzugsweise 2 bis 20 Gew.-% und insbesondere 5 bis 15 Gew.-% Ethanol oder ein beliebiges Gemisch aus Ethanol und 1,2-Propandiol oder insbesondere aus Ethanol und Glycerin. Ebenso ist es möglich, daß die Zubereitungen entweder zusätzlich zu den mono- und/oder polyfunktionellen Alkoholen mit 1 bis 6 Kohlenstoffatomen oder allein Polyethylenglykol mit einer relativen Molekülmasse zwischen 200 und 2000, vorzugsweise bis 600 in Mengen von 2 bis 17 Gew.-% enthalten. Als Hydrotrope können beispielsweise Toluolsulfonat, Xylolsulfonat, Cumolsulfonat oder deren Mischungen eingesetzt werden.

Als **Enzyme** kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Ihr Anteil kann etwa 0,2 bis etwa 2 Gew.-% betragen. Die Enzyme können an Trägerstoffen adsorbiert und/oder in Hüllsubstanzen eingebettet sein, um sie gegen vorzeitige Zersetzung zu schützen. Zusätzlich zu den mono- und polyfunktionellen Alkoholen und den Phosphonaten können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2-Gew.-%, bezogen auf das Enzym, stabilisiert sind. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B4O₇).

Als **Viskositätsregulatoren** können beispielsweise gehärtetes Rizinusöl, Salze von langkettigen Fettsäuren, die vorzugsweise in Mengen von 0 bis 5 Gew.-% und insbesondere in Mengen von 0,5 bis 2 Gew.-%, beispielsweise Natrium-, Kalium-, Aluminium-, Magnesium- und Titanstearate oder die Natrium- und/oder Kaliumsalze der Behensäure, sowie weitere polymere Verbindungen eingesetzt werden. Zu den letzteren gehören bevorzugt Polyvinylpyrrolidon, Urethane und die Salze polymerer Polycarboxylate, beispielsweise homopolymerer oder copolymerer Polyacrylate, Polymethacrylate und insbesondere Copolymere der Acrylsäure mit Maleinsäure, vorzugsweise solche aus 50 % bis 10 % Maleinsäure. Die relative Molekülmasse der Homopolymeren liegt im allgemeinen zwischen 1000 und 100000, die der Copolymeren zwischen 2000 und 200000, vorzugsweise zwischen 50000 bis 120000, bezogen auf die freie Säure. Insbesondere sind auch wasserlösliche Polyacrylate geeignet, die beispielsweise mit etwa 1 % eines Polyallylethers der Sucrose quervernetzt sind und die eine relative Molekülmasse oberhalb einer Million besitzen. Beispiele hierfür sind die unter dem Namen Carbopol® 940 und 941 erhältlichen Polymere mit verdickender Wirkung. Die quervernetzten Polyacrylate werden vorzugsweise in Mengen nicht über 1 Gew.-%, vorzugsweise in Mengen von 0,2 bis 0,7 Gew.-% eingesetzt. Die Mittel können zusätzlich etwa 5 bis 20 Gew.-% eines partiell veresterten Copolymerisats enthalten, wie es in der europäischen Patentanmeldung **EP 0367049 A** beschrieben ist. Diese partiell veresterten Polymere werden durch Copolymerisation von (a) mindestens einem C₄-C₂₈-Olefin oder Mischungen aus mindestens einem C₄-C₂₈-Olefin mit bis zu 20 Mol-% C₁-C₂₈-Alkylvinylethern und (b) ethylenisch ungesättigten Dicarbonsäureanhydriden mit 4 bis 8 Kohlenstoffatomen im Molverhältnis 1 : 1 zu Copolymerisaten mit K-Werten von 6 bis 100 und anschließende partielle Veresterung der Copolymerisate mit Umsetzungsprodukten wie C₁-C₁₃-Alkoholen, C₈-C₂₂-Fettsäuren, C₁-C₁₂₋Alkylphenolen, sekundären C₂-C₃₀-Aminen oder deren Mischungen mit mindestens einem C₂-C₄₋Alkylenoxid oder Tetrahydrofuran sowie Hydrolyse der Anhydridgruppen der Copolymerisate zu Carboxylgruppen erhalten, wobei die partielle Veresterung der Copolymerisate soweit geführt wird, daß 5 bis 50 % der Carboxylgruppen der Copolymerisate verestert sind. Bevorzugte Copolymerisate enthalten als ethylenisch ungesättigtes Dicarbonsäureanhydrid Maleinsäureanhydrid. Die partiell veresterten Copolymerisate können entweder in Form der freien Säure oder vorzugsweise in partiell oder vollständig neutralisierter Form vorliegen. Vorteilhafterweise werden die Copolymerisate in Form einer wäßrigen Lösung, insbesondere in Form einer 40 bis 50 Gew.-%igen Lösung eingesetzt. Die Copolymerisate leisten nicht nur einen Beitrag zur Primär- und Sekundärwaschleistung des flüssigen Wasch- und Reinigungsmittels, sondern bewirken auch eine gewünschte Viskositätsemiedrigung der konzentrierten flüssigen Waschmittel. Durch den Einsatz dieser partiell veresterten Copolymerisate werden konzentrierte wäßrige Flüssigwaschmittel erhalten, die unter dem alleinigen Einfluß der Schwerkraft und ohne Einwirkung sonstiger Scherkräfte fließfähig sind. Vorzugsweise beinhalten die konzentrierten wäßrigen Flüssigwaschmittel partiell veresterte Copolymerisate in Mengen von 5 bis 15 Gew.-% und insbesondere in Mengen von 8 bis 12 Gew.-%.

**Vergrauungsinhibitoren** haben die Aufgabe, den von der Faser abgelösten Schmutz in der Flotte suspendiert zu halten und so das Vergrauen zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose, Methylcellulose, Hydroxyalkylcellulose und Mischether, wie Methylhydroxyethylcellulose, Methylhydroxypropylcellulose, Methylcarboxymethylcellulose und deren Gemische sowie Polyvinylpyrrolidon, beispielsweise in Mengen von 0,1 bis 5 Gew.-%, bezogen auf die Mittel.

Die Mittel können als **optische Aufheller** Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Einheitlich weiße Granulate werden erhalten, wenn die Mittel außer den üblichen Aufhellern in üblichen Mengen, beispielsweise zwischen 0,1 und 0,5 Gew.-%, vorzugsweise zwischen 0,1 und 0,3 Gew.-%, auch geringe Mengen, beispielsweise 10⁻⁶ bis 10⁻³ Gew.-%, vorzugsweise um 10⁻⁵ Gew.-%, eines blauen Farbstoffs enthalten. Ein besonders bevorzugter Farbstoff ist Tinolux® (Handelsprodukt der Ciba-Geigy).

Als schmutzabweisenden Polymere **("soil repellants")** kommen solche Stoffe in Frage, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt insbesondere im Bereich von 750 bis 5000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglycoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease® T (ICI) oder Repelotex® SRP 3 (Rhöne-Poulenc).

Beim Einsatz in maschinellen Waschverfahren kann es von Vorteil sein, den Mitteln übliche **Schauminhibitoren** zuzusetzen. Hierfür eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, z.B. solche aus Silikonen, Paraffinen oder Wachsen. Vorzugsweise sind die Schauminhibitoren, insbesondere silikon- oder paraffinhaltige Schauminhibitoren, an eine granulare, in Wasser lösliche bzw. dispergierbare Trägersubstanz gebunden. Insbesondere sind dabei Mischungen aus Paraffinen und Bistearylethylendiamiden bevorzugt.

Der pH-**Wert** flüssiger, insbesondere auch flüssig-konzentrierter Mittel beträgt im allgemeinen 7 bis 10,5, vorzugsweise 7 bis 9,5 und insbesondere 7 bis 8,5. Die Einstellung höherer pH-Werte, beispielsweise oberhalb von 9, kann durch den Einsatz geringer Mengen an Natronlauge oder an alkalischen Salzen wie Natriumcarbonat oder Natriumsilicat erfolgen. Die flüssigen Zubereitungen weisen im allgemeinen Viskositäten zwischen 150 und 10000 mPas (Brookfield-Viskosimeter, Spindel 1, 20 Umdrehungen pro Minute, 20°C) auf. Dabei sind bei den im wesentlichen wasserfreien Mitteln Viskositäten zwischen 150 und 5000 mPas bevorzugt. Die Viskosität dieser wäßrigen Mittel liegt vorzugsweise unter 2000 mPas und liegt insbesondere zwischen 150 und 1000 mPas.

### Herstellung fester Zubereitungen

Das Schüttgewicht der fester Zubereitungen beträgt im allgemeinen 300 bis 1200 g/l, insbesondere 500 bis 1100 g/l. Ihre Herstellung kann nach jedem der bekannten Verfahren wie Mischen, Sprühtrocknung, Granulieren und Extrudieren erfolgen. Geeignet sind insbesondere solche Verfahren, in denen mehrere Teilkomponenten, beispielsweise sprühgetrocknete Komponenten und granulierte und/oder extrudierte Komponenten miteinander vermischt werden. Dabei ist es auch möglich, daß sprühgetrocknete oder granulierte Komponenten nachträglich in der Aufbereitung beispielsweise mit nichtionischen Tensiden, insbesondere ethoxylierten Fettalkoholen, nach den üblichen Verfahren beaufschlagt werden. Insbesondere in Granululations- und Extrusionsverfahren ist es bevorzugt, die gegebenenfalls vorhandenen Aniontenside in Form eines sprühgetrockneten, granulierten oder extrudierten Compounds entweder als Zumischkomponente in dem Verfahren oder als Additiv nachträglich zu anderen Granulaten einzusetzen. Insbesondere die bevorzugten schwereren Granulate mit Schüttgewichten oberhalb 600 g/l enthalten vorzugsweise Komponenten, welche das Einspülverhalten und/oder das Löseverhalten der Granulate verbessem. Vorteilhafterweise werden hierzu alkoxylierte Fettalkohole mit 12 bis 80 Mol Ethylenoxid pro Mol Alkohol, beispielsweise Talgfettalkohol mit 14 EO, 30 EO oder 40 EO, und Polyethylenglykole mit einer relativen Molekülmasse zwischen 200 und 12000, vorzugsweise zwischen 200 und 600, eingesetzt.

Ebenso ist es möglich und kann in Abhängigkeit von der Rezeptur von Vorteil sein, wenn weitere einzelne Bestandteile des Mittels, beispielsweise Citrat bzw. Citronensäure oder andere Polycarboxylate bzw. Polycarbonsäuren, polymere Polycarboxylate, Zeolith und/oder Schichtsilikate, die gegebenenfalls kristallin sein können, nachträglich zu sprühgetrockneten, granulierten und/oder extrudierten Komponenten, die gegebenenfalls mit nichtionischen Tensiden und/oder anderen bei der Verarbeitungstemperatur flüssigen bis wachsartigen Inhaltsstoffen beaufschlagt sind, hinzugemischt werden. Bevorzugt ist dabei ein Verfahren, bei dem die Oberfläche von Teilkomponenten des Mittels oder des gesamtem Mittels zur Reduzierung der Klebrigkeit der an Niotensiden reichen Granulate und/oder zu ihrer verbesserten Löslichkeit nachträglich behandelt wird. Geeignete Oberflächenmodifizierer sind dabei aus dem Stand der Technik bekannt. Neben weiteren geeigneten sind dabei feinteilige Zeolithe, Kieselsäuren, amorphe Silikate, Fettsäuren oder Fettsäuresalze, beispielsweise Calciumstearat, insbesondere jedoch Mischungen aus Zeolith und Kieselsäuren oder Zeolith und Calciumstearat besonders bevorzugt.

### Beispiele

Die **Hautverträglichkeit** der Detergensgemische wurde entsprechend der OECD-Methode No.404 und der EEC Directive 84/449 EEC, Pt.B.4. bestimmt. Die angegebenen Reizsummenscores wurden aus den nach 24, 48 und 72 h erhaltenen Reizscores gebildet. Dabei wurde der im Vergleichsversuch V1 ermittelte Reizsummenscore zu 100 % gesetzt und die in den übrigen Versuchen erhaltenen Scores zu diesem ins Verhältnis gesetzt. Die Beurteilung des **Weichgriffs** erfolgte durch ein Panel von 6 geschulten Personen, die die gewaschenen Baumwollgewebe auf einer Skala von (1) = sehr weich bis (4) = hart bewerteten. Die **Hydrophilie**, d.h. Wiederbenetzbarkeit der Gewebe, wurde im bekannten Steighöhentest nach DIN 53924 bestimmt, bei dem man Streifen des Baumwollgewebes von 1 cm Breite in Wasser eintaucht und die Höhe mißt, auf die das Wasser aufgrund der Kapillarkräfte in dem Gewebe innerhalb von 1 min steigt; je größer die Steighöhe um so höher ist auch die Hydrophilie des Gewebes.

**Tabelle 1 Zusammensetzung und Performance von Detergensgemischen**

| **Zusammensetzung / Performance** | **1** | **2** | **3** | **4** | **5** | **6** | **7** | **8** | **V1** | **V2** |
|---|---|---|---|---|---|---|---|---|---|---|
| Esterquat* | 35,0 | 35,0 | 30,0 | 30,0 | 30,0 | 30,0 | 30,0 | 30,0 | - | - |
| Distearyldimethylammonium chloride | - | - | - | - | - | - | - | - | 35,0 | 35,0 |
| Aloe vera | 1,0 | - | 1,0 | 1,0 | 1,0 | - | - | - | - | 1,0 |
| Aloe ferox | - | 1,0 | - | - | - | 1,0 | 1,0 | 1,0 | - | - |
| Coco Glucosides | - | - | 5,0 | - | 3,0 | 5,0 | - | 3,0 | - | - |
| Cocamidopropyl Betain | - | - | - | 5,0 | 2,0 | - | 5,0 | 2,0 | - | - |
| Wasser | ad 100 | | | | | | | | | |
| ***Reizsummenscore [%-rel]*** | 84 | 85 | 80 | 76 | 72 | 80 | 77 | 72 | 100 | 90 |
| ***Weichgriff*** | 1,5 | 1,5 | 1,5 | 1,5 | 1,5 | 1,5 | 1,5 | 1,5 | 2,5 | 2,5 |
| ***Hydrophilie [mm]*** | 11 | 12 | 12 | 12 | 11 | 12 | 11 | 12 | 8 | 9 |

| | | | | | | | | | | |
|---|---|---|---|---|---|---|---|---|---|---|
| *) Methylquaternierter Ditalgfettsäuretriethanolaminester, Methylsulfat-Salz (Dehyquart AU 46, Henkel KGaA, Düsseldorf) | | | | | | | | | | |

**Tabelle 2 Zusammensetzung und Performance von Detergensgemischen**

| **Zusammensetzung / Performance** | **9** | **10** | **11** | **12** | **13** | **14** |
|---|---|---|---|---|---|---|
| Esterquat 1 | 35,0 | - | - | - | - | - |
| Esterquat 2 | - | 35,0 | - | - | - | - |
| Esterquat 3 | - | - | 35,0 | - | - | - |
| Esterquat 4 | - | - | - | 35,0 | - | - |
| Esterquat 5 | - | - | - | - | 35,0 | - |
| Esterquat 6 | - | - | - | - | - | 35,0 |
| Aloe vera | 1,0 | 1,0 | 1,0 | 1,0 | 1,0 | 1,0 |
| Wasser | ad 100 | | | | | |
| ***Reizsummenscore [%-rel]*** | 85 | 83 | 84 | 85 | 82 | 83 |
| ***Weichgriff*** | 1,5 | 2,0 | 2,0 | 2,0 | 2,0 | 2,0 |
| ***Hydrophilie [mm]*** | 11 | 10 | 10 | 10 | 10 | 10 |

| | | | | | | |
|---|---|---|---|---|---|---|
| (1) Dipalmitoylethyl Hydroxyethylmonium Methosulfate (Dehyquart AU 56) | | | | | | |
| (2) Dipalmitoylethyl Hydroxyethylmonium Methosulfate (and) Ceteryl Alcohol (and) Ceteareth-20 (Dehyquart C 4043) | | | | | | |
| (3) Dipalmitoyl/adipinolyethyl Hydroxyethylmonium Methosulfate (Dehyquart D 6003) | | | | | | |
| (4) Dipalmitoylethyl Hydroxyethylmonium Methosulfate (and) Ceteryl Alcohol (Dehyquart F 75) | | | | | | |
| (5) Dipalmitoylethyl Hydroxyethylmonium Methosulfate (and) Cocoglycerides (and) Glycerin (Dehyquart F 100) | | | | | | |
| (6) Dicocoylethyl Hydroxyethylmonium Methosulfate (and) Propylenglycol (Dehyquart L 80) - alle Henkel KGaA, Düsseldorf | | | | | | |

## Patentansprüche

1. Verwendung von Detergensgemischen, enthaltend
(a) Esterquats,
(b) Aloe
zur Herstellung von Avivagemitteln.

2. Verwendung von Detergensgemischen nach Anspruch 1, **dadurch gekennzeichnet, daß** sie Esterquats der Formel **(I)** enthalten, 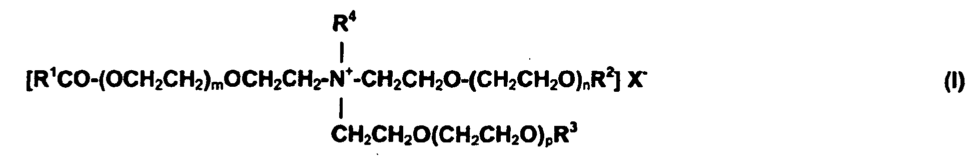 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Verwendung von Detergensgemischen nach Anspruch 1, **dadurch gekennzeichnet, daß** sie Esterquats der Formel **(II)** enthalten, 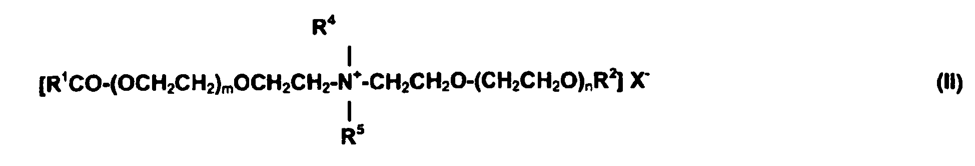 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verwendung von Detergensgemischen nach Anspruch 1, **dadurch gekennzeichnet, daß** sie Esterquats der Formel **(III)** enthalten, 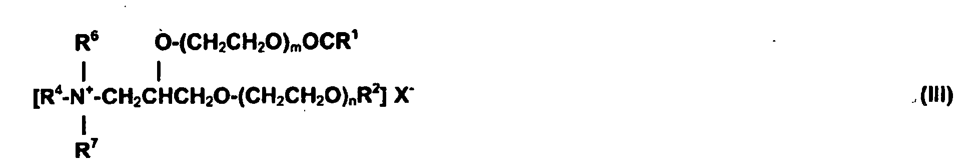 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung von Detergensgemischen nach mindestens einem der Ansprüche 1 bis 4, **dadurch gekennzeichnet, daß** sie Aloe vera enthalten.

6. Verwendung von Detergensgemischen nach mindestens einem der Ansprüche 1 bis 5, **dadurch gekennzeichnet, daß** sie weitere Tenside enthalten.

7. Verwendung von Detergensgemischen nach mindestens einem der Ansprüche 1 bis 6, **dadurch gekennzeichnet, daß** sie
(a) 1 bis 50 Gew.-% Esterquats,
(b) 1 bis 10 Gew.-% Aloe und
(c) 0 bis 25 Gew.-% weitere Tenside
mit der Maßgabe enthalten, daß sich die Gewichtsangaben mit Wasser sowie gegebenenfalls weiteren Hilfs- und Zusatzstoffen zu 100 Gew.-% ergänzen.

## Claims

1. Use of detergent mixtures containing
(a) esterquats
(b) aloe
for the production of softeners.

2. Use of detergent mixtures as claimed in claim 1, **characterized in that** they contain esterquats corresponding to formula **(I)**: 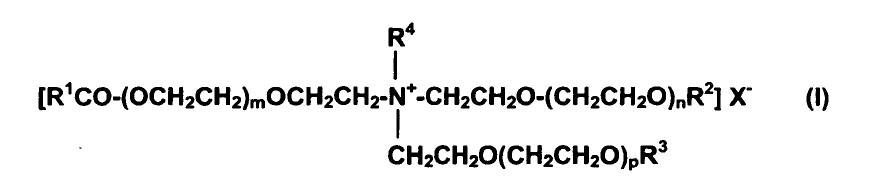 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate.

3. Use of detergent mixtures as claimed in claim 1, **characterized in that** they contain esterquats corresponding to formula **(II):** 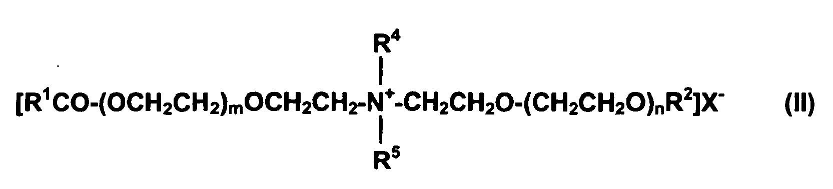 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

4. Use of detergent mixtures as claimed in claim 1, **characterized in that** they contain esterquats corresponding to formula **(III):** 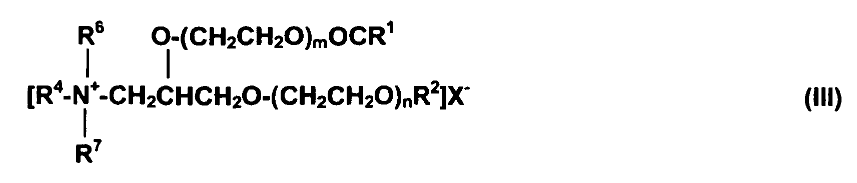 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

5. Use of detergent mixtures as claimed in at least one of claims 1 to 4, **characterized in that** they contain aloe vera.

6. Use of detergent mixtures as claimed in at least one of claims 1 to 5, **characterized in that** they contain other surfactants.

7. Use of detergent mixtures as claimed in at least one of claims 1 to 6, **characterized in that** they contain
(a) 1 to 50% by weight esterquats,
(b) 1 to 10% by weight aloe and
(c) 0 to 25% by weight of other surfactants,
with the proviso that the percentages by weight add up to 100% by weight with water and optionally other auxiliaries and additives.

## Revendications

1. Utilisation de mélanges détergents contenant
(a) des esters d'ammonium quaternaire,
(b) de l'aloé
pour fabriquer des agents d'avivage.

2. Utilisation de mélanges détergents selon la revendication 1,
**caractérisée ce qu'**
ils contiennent des esters d'ammonium quaternaire de formule (I), 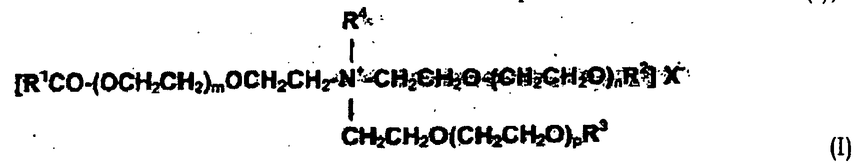 dans laquelle R¹CO représente un radical acyle portant 6 à 22 atomes de carbone, R² et R³, représentent indépendamment l'un de l'autre, un atome d'hydrogène, ou R¹CO, R⁴ représente un radical alkyle portant 1 à 4 atomes de carbone ou un groupe (CH₂CH₂O)_{q}H, m, n et p ont pour somme 0 ou des nombres de 1 à 12, q est un nombre de 1 à 12, et X représente un halogénure, un sulfate d'alkyle ou un phosphate d'alkyle.

3. Utilisation de mélanges détergents selon la revendication 1,
**caractérisée en ce qu'**
ils contiennent des esters d'ammonium quaternaire de formule (II) 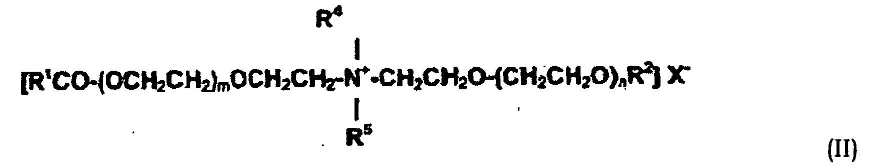 dans laquelle R¹CO représente un radical acyle portant 6 à 22 atomes de carbone, R² représente un atome d'hydrogène ou R¹CO, R⁴ et R⁵ représentent indépendamment l'un de l'autre, des radicaux alkyle portant 1 à 4 atomes de carbone, m et n ont pour somme, 0 ou des nombres de 1 à 12, et X représente un halogénure, un sulfate d'alkyle ou un phosphate d'alkyle.

4. Utilisation de mélanges détergents selon la revendication 1,
**caractérisée en ce qu'**
ils contiennent des esters d'ammonium quaternaire de formule III, 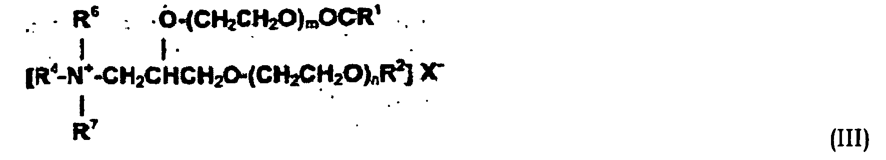 dans laquelle R¹CO représente un radical acyle portant 6 à 22 atomes de carbone, R² représente un atome d'hydrogène ou R¹CO ; R⁴, R⁶ et R⁷ représentent indépendamment les uns des autres des radicaux alkyle portant 1 à 4 atomes de carbone, m et n, ont pour somme O ou des nombres de 1 à 12, et X représente un halogénure, un sulfate d'alkyle ou un phosphate d'alkyle.

5. Utilisation de mélanges détergents selon au moins l'une quelconque des revendications 1 à 4,
**caractérisée en ce qu'**
ils contiennent de l'aloé vera.

6. Utilisation de mélanges détergents selon au moins l'une quelconque des revendications 1 à 5,
**caractérisée en ce qu'**
ils contiennent d'autres agents tensioactifs.

7. Utilisation de mélanges détergents selon au moins l'une quelconque des revendications 1 à 6,
**caractérisée en ce qu'**
ils contiennent
(a) 1 à 50 % en poids d'esters d'ammonium quaternaire,
(b) 1 à 10 % en poids d'aloé et
(c) 0 à 25 % en poids d'autres agents tensioactifs
les poids indiqués se complètant avec de l'eau ainsi que, le cas échéant d'autres excipients et additifs, pour donner 100 % en poids.
